# EUROPEAN PATENT APPLICATION

(11) **EP 3 494 884 A1**
(43) Date of publication of application: **12.06.2019**
(21) Application number: 16911608.4
(22) Date of filing: 03.08.2016
(51) Int. Cl.: A61B 6/00

(54) **X-RAY FLUOROSCOPIC IMAGING APPARATUS**

(71) Applicant: Shimadzu Corporation, Kyoto-shi, Kyoto 604-8511 (JP)
(72) Inventor: SAKAMOTO, Yuki, Kyoto-shi Kyoto 604-8511 (JP); OKAMOTO, Takeshi, Kyoto-shi Kyoto 604-8511 (JP)
(74) Representative: Becker Kurig Straus
(86) International application number: PCT/JP2016/072794
(87) International publication number: WO 2018/025347

(57) **Abstract**

In the present invention, a control unit 10 is provided with a region of interest setting unit 21, an irradiation condition automatic adjustment unit 22, and a site of interest tracking unit 23. This X-ray fluoroscopic imaging apparatus makes automatic adjustments to correct X-ray irradiation conditions on the basis of luminance of a region of interest in an X-ray image so that a site of interest to be observed such as a specific organ or lesion of a subject 4 can be suitably viewed. When the site of interest moves outside the set region of interest, the site of interest tracking unit 23 tracks the movement of the site of interest and resets the region of interest so as to include the site of interest.

## Description

### [Technical Field]

The present invention relates to an X-ray fluoroscopic imaging apparatus which carries out X-ray fluoroscopy by detecting X-rays radiated from an X-ray tube and transmitted through a subject with an X-ray detector.

### [Background Art]

An X-ray fluoroscopic imaging apparatus is an apparatus which allows a doctor to perform image diagnosis by irradiating a subject placed on a top plate with X-rays, detecting the X-rays which have been transmitted through the subject and then imaging the X-rays. As an apparatus of this type, there has been proposed an X-ray apparatus which adjusts X-ray irradiation conditions during X-ray fluoroscopy and automatically adjusts a luminance of a fluoroscopic image displayed on an image display apparatus such that it becomes suitable for observation (refer to Patent document 1). Further, an X-ray image diagnostic apparatus which sets a region of interest (ROI) surrounding a site (a site of interest) such as a specific organ or lesion which is desired to be carefully observed on a fluoroscopic image and sets X-ray conditions on the basis of luminance information of the region of interest has been proposed (refer to Patent Document 2).

### [Citation List]

### [Patent Literature]

[Patent Literature 1]
   Japanese Patent Application Laid-Open No. 2000-261724
[Patent Literature 2]
   Japanese Patent Application Laid-Open No. 2015-228994

### [Summary of Invention]

### [Technical Problem]

During the X-ray fluoroscopy, a posture of the subject lying on the top plate may change, and a position of the site of interest such as a specific organ or lesion on a display screen may deviate from the set region of interest. Then, even when the X-ray conditions are set on the basis of the luminance information of the region of interest, appropriate luminance adjustment for the site of interest may not be performed, and visibility of the site of interest may be degraded. Therefore, in Patent Document 2, when the site (the site of interest) desired to be checked is brought outside the region of interest due to movement of the subject or the like, the region of interest is set according to movement of an operator's sight line.

However, in adopting a sight line detection sensor, it is necessary to consider a distance between the eyes and the sight line detection sensor, a state of illumination in a place in which the apparatus will be used, and so on. Also, when the X-ray fluoroscopy is performed during a surgical operation of a patient for the purpose of assisting the progress of the surgical operation, many people may observe the X-ray image displayed on a monitor, and thus it is necessary to consider whose sight line as a priority to set the region of interest.

The present invention has been made to solve the above problems, and an objective thereof is to provide an X-ray fluoroscopic imaging apparatus capable of displaying an image with an appropriate luminance even when a site of interest moves.

### [Solution to Problem]

According to the present invention described in claim 1, there is provided an X-ray fluoroscopic imaging apparatus including an X-ray tube which generates X-rays to be radiated to a subject, an X-ray detector which detects the X-rays radiated from the X-ray tube and passing through the subject, an image generation unit which generates an X-ray image by processing a detection signal of the X-ray detector and displays the X-ray image on a display unit, and a control unit having a processor, wherein the control unit includes a region of interest setting unit which sets a region of interest on the X-ray image, an irradiation condition automatic adjustment unit which calculates X-ray irradiation conditions on the basis of a set luminance of the region of interest, and a site of interest tracking unit which tracks movement of a site of interest, and the region of interest setting unit resets the region of interest according to the movement of the site of interest tracked by the site of interest tracking unit.

According to the present invention described in claim 2, in the invention described in claim 1, the site of interest tracking unit may extract a feature amount of a region set as the region of interest, may distinguish the movement of the site of interest from a change in the feature amount and may search for a region having a feature amount closest to the feature amount in the X-ray image, and the region of interest setting unit may reset a region having a feature amount closest to the feature amount in the X-ray image identified by the site of interest tracking unit to the region of interest.

According to the present invention described in claim 3, in the invention described in claim 1, the control unit may include a storage unit which temporarily stores movement information when a display orientation of the X-ray image displayed on the display unit is changed, the site of interest tracking unit may calculate a movement position of the site of interest in the X-ray image on the basis of the movement information of the X-ray image acquired from the storage unit, and the region of interest setting unit may reset a region on the X-ray image including the site of interest, of which a position is calculated by the site of interest tracking unit, to the region of interest.

According to the present invention described in claim 4, in the invention described in claim 1, the control unit may include a storage unit which temporarily stores movement information when a position of the X-ray detector is changed, the site of interest tracking unit may calculate a movement position of the site of interest in the X-ray image on the basis of the movement information of the X-ray detector acquired from the storage unit, and the region of interest setting unit may reset a region on the X-ray image including the site of interest, of which a position is calculated by the site of interest tracking unit, to the region of interest.

According to the present invention described in claim 5, in the X-ray fluoroscopic imaging apparatus described in claim 1, the control unit may include a mode switching unit which receives an input from an operation button and switches between execution and stop of a tracking operation of the site of interest by the site of interest tracking unit.

### [Advantageous Effects of Invention]

According to the invention described in claims 1 to 5, even when the site of interest which is a site desired to be carefully observed is outside the region of interest, since the movement of the site of interest is tracked by the site of interest tracking unit and the region of interest is reset to include the site of interest, it is possible to adjust an image luminance with which a visibility of the site of interest M can be maintained in a preferable state.

According to the invention described in claim 2, since the site of interest tracking unit extracts the feature amount of the region of interest including the site of interest and identifies a region on the X-ray image in which the site of interest has moved using this feature amount, tracking of the site of interest can be performed accurately even when a posture of the subject changes during X-ray fluoroscopy.

According to the invention described in claim 3, since the site of interest tracking unit calculates the position on the X-ray image in which the site of interest has moved using the movement information when a display orientation of the X-ray image displayed on the display unit is changed, the resetting of the region of interest can be performed promptly even when the X-ray image is rotated and moved in a direction in which the operator can easily see the X-ray image.

According to the invention described in claim 4, since the site of interest tracking unit calculates a position on the X-ray image in which the site of interest has moved using the movement information when a position of the X-ray detector is changed, the resetting of the region of interest can be performed promptly even when the X-ray detector is moved during the X-ray fluoroscopy.

According to the invention described in claim 5, since there is provided the mode switching unit which receives an input from the operation button and switches between execution and stop of the tracking operation of the site of interest by the site of interest tracking unit, the operator can select to execute the tracking of the site of interest as necessary.

### [Brief Description of Drawings]

FIG. 1 is a schematic diagram showing a main configuration of an X-ray fluoroscopic imaging apparatus according to the present invention.
FIG. 2 is a block diagram showing a functional configuration of a control unit 10 according to a first embodiment.
FIG. 3 is an explanatory diagram showing a movement of a site of interest M and setting of a region of interest R.
FIG. 4 is an explanatory diagram showing the movement of the site of interest M and the setting of the region of interest R.
FIG. 5 is a flowchart showing a tracking operation of the site of interest M in a site of interest tracking unit 23.
FIG. 6 is a flowchart showing a modified example of the tracking operation of the site of interest M in the site of interest tracking unit 23.
FIG. 7 is a schematic diagram of the X-ray fluoroscopic imaging apparatus in which an X-ray tube 1 and an X-ray detector 2 are supported by a C arm 32.
FIG. 8 is a block diagram showing a functional configuration of a control unit 10 of an X-ray fluoroscopic imaging apparatus according to a second embodiment.

### [Description of Embodiments]

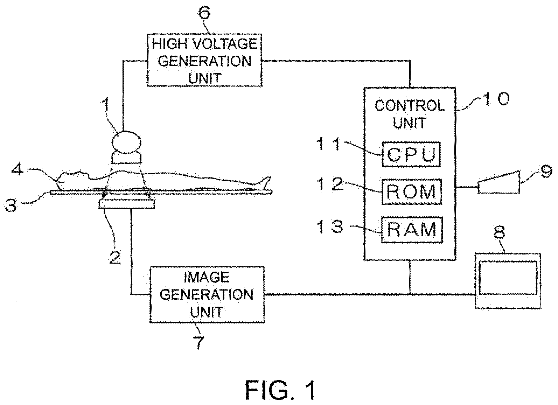

Hereinafter, embodiments of the present invention will be described with reference to the drawings. FIG. 1 is a schematic diagram showing a main configuration of an X-ray fluoroscopic imaging apparatus according to the present invention.

The X-ray fluoroscopic imaging apparatus includes a top plate 3 on which a subject 4 in a supine posture is placed, an X-ray tube 1 which irradiates X-rays toward the subject 4, an X-ray detector 2 which detects the X-rays passing through the subject 4, and a control unit 10 which has a processor (a CPU 11) and controls the entire apparatus. In this X-ray fluoroscopic imaging apparatus, fluoroscopy for continuously observing the subject 4 is performed by displaying fluoroscopic images of the subject 4 on a display unit 8 such as a liquid crystal display panel.

The control unit 10 includes a CPU 11 which executes various calculations, a ROM 12 as a storage unit, and a RAM 13. The ROM 12 stores a program or the like necessary for an operation, and the RAM 13 temporarily stores data such as X-ray photographing conditions or control amounts when executing various operations. The control unit 10 is connected to the display unit 8 and an operation unit 9 which receives an input from an operator. A keyboard, a mouse, a touch panel disposed on the display unit 8, or the like can be used as the operation unit 9. Further, the control unit 10 is also connected to the X-ray tube 1 via a high voltage generation unit 6. The high voltage generation unit 6 supplies a tube voltage/tube current suitable for X-ray fluoroscopy and photographing to the X-ray tube 1 using a control signal from control unit 10.

The control unit 10 is connected to the X-ray detector 2 via an image generation unit 7. The X-ray detector 2 detects X-rays radiated from the X-ray tube 1 and passing through the subject 4, converts the X-rays into an electrical signal and outputs an X-ray detection signal. The image generation unit 7 generates an image by processing the detection signal of the X-ray detector 2 and displays the image on the display unit 8. An image intensifier (I.I.) or a flat panel detector (FPD) can be used as the X-ray detector 2.

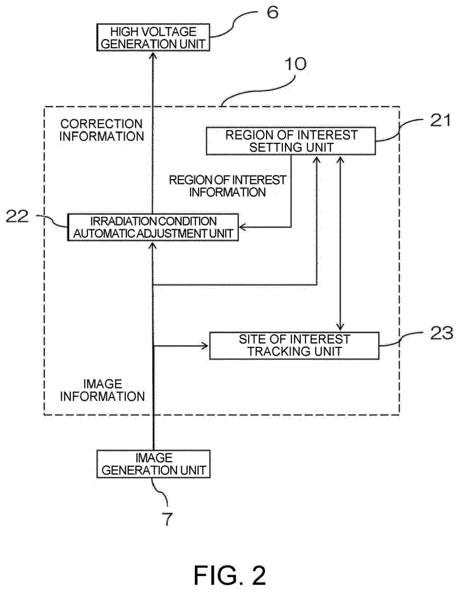

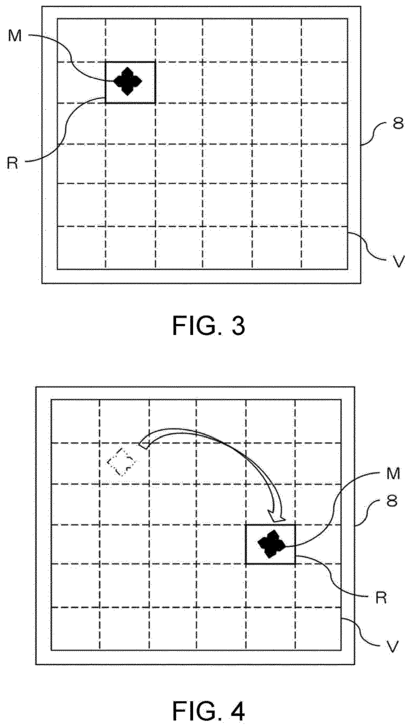

FIG. 2 is a block diagram for explaining a functional configuration of the control unit 10 according to the first embodiment. FIGS. 3 and 4 are explanatory diagrams showing movement of a site of interest M and setting of a region of interest R. FIG. 3 shows the region of interest R arbitrarily set by an operator, and FIG. 4 shows resetting of the region of interest R due to the movement of the site of interest M. In FIGS. 3 and 4, a field of view V when the flat panel detector (FPD) is employed as the X-ray detector 2 is shown, but in the case of the image intensifier (I.I.), the field of view V is circular.

The control unit 10 of the embodiment includes a region of interest setting unit 21, an irradiation condition automatic adjustment unit 22, and a site of interest tracking unit 23. In the X-ray fluoroscopic imaging apparatus, automatic adjustment for correcting X-ray irradiation conditions is performed on the basis of a luminance value of the region of interest R in an X-ray image so that a site of interest M desired to be observed such as a specific organ or lesion of the subject 4 can be suitably viewed. Various calculations in the region of interest setting unit 21, the irradiation condition automatic adjustment unit 22 and the site of interest tracking unit 23 are executed by the CPU 11.

When X-ray fluoroscopy with respect to the subject 4 is started and the X-ray image is displayed on the display unit 8, the operator operates the operation unit 9 and designates the region of interest R on a screen in accordance with a position of the site of interest M. When the operator designates the region of interest R, a grid for dividing the image into a plurality of rectangular regions is displayed on the X-ray image, and the region of interest setting unit 21 sets one or more regions arbitrarily designated by the operator among the separate regions on the X-ray image as the region of interest R (refer to FIG. 3). The number of separate regions on the screen of the display unit 8 can be appropriately changed by the operator operating the operation unit 9. Since the display of the region of interest R is realized by superimposing a grid for dividing the image shown in FIGS. 3 and 4 or a layer for drawing a setting range of the region of interest R on the X-ray image, even when the X-ray image is updated at predetermined intervals during the X-ray fluoroscopy, the setting range of the region of interest R drawn on the layer different from the X-ray image does not move. Therefore, when the site of interest M in the updated X-ray image deviates from the region of interest R, it is necessary to track the site of interest M and to move the region of interest R.

When the region of interest setting unit 21 sets the region of interest R, the region of interest setting unit 21 sends region of interest information including the luminance value of each pixel of the set region of interest R to the irradiation condition automatic adjustment unit 22. In the irradiation condition automatic adjustment unit 22, the image luminance value of the region of interest R is compared with a predetermined ideal luminance value, and a correction value for correcting the X-ray irradiation conditions is calculated so that the luminance value of the X-ray image becomes the ideal luminance value. Here, the image luminance value which is compared with the ideal luminance value is, for example, an average value of the luminance values of the respective pixels located within the region of interest R. The correction value calculated by the irradiation condition automatic adjustment unit 22 is sent as correction information to the high voltage generation unit 6, and thus the tube voltage/tube current supplied to the X-ray tube 1 is changed. As a result of the X-ray irradiation under the corrected X-ray irradiation conditions, the luminance value of the X-ray image generated by the image generation unit 7 becomes the ideal luminance value. In this X-ray fluoroscopic imaging apparatus, such X-ray irradiation conditions are automatically adjusted for the X-ray images intermittently generated by the X-ray fluoroscopy.

When the posture of the subject 4 changes during the X-ray fluoroscopy, the site of interest M may move out of the region of interest R set by the region of interest setting unit 21. Therefore, in this X-ray fluoroscopic imaging apparatus, the movement of the site of interest M is tracked by an action of the site of interest tracking unit 23, and the region of interest R is moved so that the site of interest M which has moved from a position indicated by an imaginary line in FIG. 4 is always included in the region of interest R.

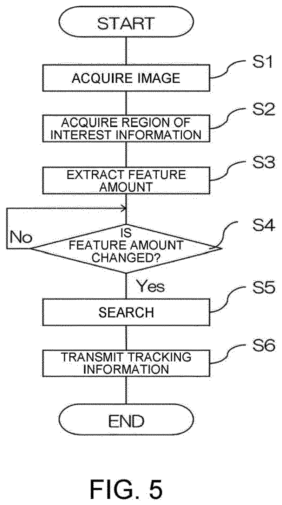

FIG. 5 is a flowchart showing a tracking operation of the site of interest M in the site of interest tracking unit 23.

The site of interest tracking unit 23 acquires the image information from the image generation unit 7 (Step S1) and acquires the region of interest information from the region of interest setting unit 21 (Step S2). This region of interest information includes the position and the luminance value of the respective pixels on the image of the region of interest R set to include the site of interest M. Thereafter, the site of interest tracking unit 23 extracts a feature amount of the region of interest R (Step S3).

Here, information such as a proportion occupied by the site of interest M in the region of interest R, the luminance value of the respective pixels in the region of interest R, a shape of the site of interest M, and a contrast between the site of interest M and the other regions in the region of interest R can be used as the feature amount of the region of interest R. When the site of interest M deviates from the region of interest R due to a change in the posture of the subject 4, for example, a luminance difference equal to or greater than a predetermined value which is present at a boundary between the site of interest M and the other regions in the region of interest R is not observed. In this way, information which can determine the presence or absence of the site of interest M in the region of interest R can be adopted as the feature amount.

Next, it is determined whether there is a change in the feature amount of the region of interest R (Step S4). This determination is repeatedly performed at predetermined time intervals. When there is the change in the feature amount, it is determined that the site of interest M has moved outside the region of interest R, and the site of interest tracking unit 23 searches for a region closest to the previously extracted feature amount in the current X-ray image displayed on the display unit 8 (Step S5). In the embodiment, since the image is divided by the grid, for example, the search can be performed by determining similarity with the feature amount of the region of interest R for each divided region. As a result of the search, when a region having a feature amount closest to the previously extracted feature amount is identified, the site of interest tracking unit 23 sends the information as tracking information to the region of interest setting unit 21 (Step S6).

The region of interest setting unit 21 receives the tracking information sent from the site of interest tracking unit 23 and resets the region having the feature amount closest to the previously extracted feature amount to the region of interest R. The region of interest information including a luminance value of the respective pixels of the reset region of interest R is sent to the irradiation condition automatic adjustment unit 22. In the irradiation condition automatic adjustment unit 22, the luminance value of the reset region of interest R is compared with a predetermined ideal luminance value, and a correction value for correcting the X-ray irradiation conditions is calculated so that the luminance value of the site of interest M in the X-ray image becomes the ideal luminance value. Additionally, the correction value calculated by the irradiation condition automatic adjustment unit 22 is sent as correction information to the high voltage generation unit 6, and thus the tube voltage/tube current supplied to the X-ray tube 1 is changed.

The tracking of the site of interest M in the site of interest tracking unit 23 described with reference to FIG. 5 is repeatedly performed whenever the region of interest R is reset. In this way, it is possible to adjust the image luminance with which a visibility of the site of interest M can be maintained in a preferable state by changing the setting of the region of interest R in accordance with the movement of the site of interest M.

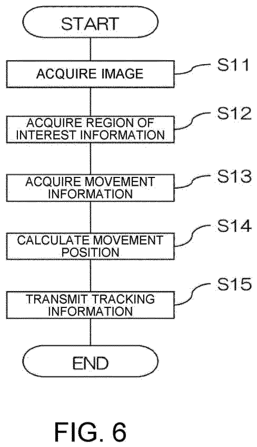

Next, a modified example of tracking the site of interest M in the site of interest tracking unit 23 will be described. FIG. 6 is a flowchart showing a modified example of the tracking operation of the site of interest M in the site of interest tracking unit 23.

In the tracking of the site of interest M described with reference to FIG. 5, the feature amount extracted from the X-ray image is used, but in this modified example, the site of interest M is tracked in accordance with a rotation angle or a movement amount when the operator rotates and moves the image by operating the operation unit 9 with respect to the X-ray image displayed on the display unit 8

When the X-ray fluoroscopy is performed during surgery of a patient for the purpose of assisting the progress of a surgical operation, it may be required to change a direction of the X-ray image displayed on the display unit 8 according to a positional relationship between a doctor who is a surgeon and the patient. In the case in which the apparatus is configured so that the X-ray image displayed on the display unit 8 can be rotated and/or moved upward, downward, right and left on the screen in response to such a request, such movement information is temporarily stored in the RAM 13 of the control unit 10.

The site of interest tracking unit 23 acquires the image information from the image generation unit 7 (Step S11), acquires the region of interest information from the region of interest setting unit 21 (Step S12) and acquires the movement information of the X-ray image (Step S13). This movement information includes information such as the rotation angle when the X-ray image is rotated and the movement amount when the X-ray image is moved upward, downward, right and left. A movement position of the site of interest M on the screen is calculated using this movement information (Step S14). When a region expected to include the site of interest M is identified from the calculation result of the movement position, the site of interest tracking unit 23 sends the information as tracking information to the region of interest setting unit 21 (Step S15).

The region of interest setting unit 21 receives the tracking information sent from the site of interest tracking unit 23 and resets the region of interest R. The region of interest information including the luminance value of each pixel of the reset region of interest R is sent to the irradiation condition automatic adjustment unit 22. In the irradiation condition automatic adjustment unit 22, the luminance value of the reset region of interest R is compared with a predetermined ideal luminance value, and a correction value for correcting the X-ray irradiation conditions is calculated so that the luminance value of the X-ray image becomes the ideal luminance value. Additionally, the correction value calculated by the irradiation condition automatic adjustment unit 22 is sent as correction information to the high voltage generation unit 6, and thus the tube voltage/tube current supplied to the X-ray tube 1 is changed. In this way, it is possible to adjust the image luminance with which the visibility of the site of interest M can be maintained in a preferable state by changing the setting of the region of interest R in accordance with the movement of the site of interest M.

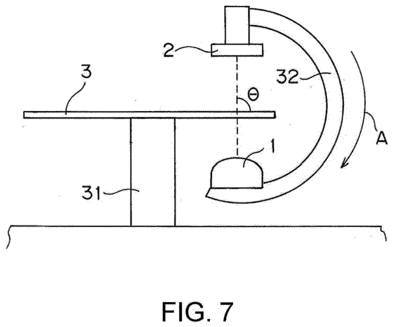

Another modified example of tracking the site of interest M in the site of interest tracking unit 23 will be described. FIG. 7 is a schematic diagram of the X-ray fluoroscopic imaging apparatus in which the X-ray tube 1 and the X-ray detector 2 are supported by the C arm 32.

In this modified example, assuming that the apparatus is an apparatus capable of changing positions of the X-ray tube 1 and the X-ray detector 2 during the X-ray fluoroscopy, the site of interest M is tracked according to the rotation angle or the movement amount when the X-ray detector 2 is rotated and moved. As the apparatus capable of changing the positions of the X-ray tube 1 and the X-ray detector 2 during the X-ray fluoroscopy, there is the X-ray fluoroscopic imaging apparatus in which the X-ray tube 1 and the X-ray detector 2 shown in FIG. 7 are supported by the C arm 32.

For example, when the C arm 32 is slid in an A direction, an intersection angle θ of an X-ray axis from the X-ray tube 1 to the X-ray detector 2 with respect to the top plate 3 horizontally supported by a support fixture 31 changes. In the apparatus capable of changing the positions of the X-ray tube 1 and the X-ray detector 2 in X-ray fluoroscopy, the rotation angle/movement amount of the X-ray detector 2 from the rotation angle/movement amount when the C arm 32 is slid and rotated are temporarily stored as a position control amount or the like in the RAM 13 of the control unit 10.

The tracking of the site of interest M in the site of interest tracking unit 23 in this modified example is different from the above-described modified example in the details of the movement information in Step S13 in the procedure shown in FIG. 5. That is, in this modified example, the movement information acquired in Step S13 is replaced with the movement information of the X-ray detector 2 from the movement information of the X-ray image. Also, the movement information of the X-ray detector 2 includes information such as the rotation angle, the movement amount, the movement direction, and the intersection angle θ between the top plate 3 and the X-ray axis when the X-ray detector 2 is moved.

In this modified example, since the site of interest M is tracked according to the movement of the X-ray detector 2 and the region of interest R is changed, even when the site of interest M is brought outside the region of interest R due to changing of an arrangement of the X-ray imaging system including the X-ray tube 1 and the X-ray detector 2, the region of interest R can be promptly set to include the site of interest M, and thus an appropriate image luminance can be maintained.

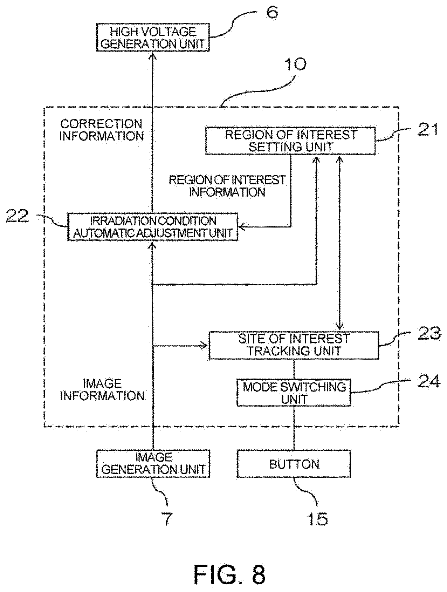

FIG. 8 is a block diagram showing a functional configuration of a control unit 10 of an X-ray fluoroscopic imaging apparatus according to a second embodiment. The same reference numerals are given to components which are the same as those of the first embodiment, and detailed description thereof will be omitted.

In the embodiment, a mode switching unit 24 is provided in the control unit 10 so that the operation of the site of interest tracking unit 23 can be stopped when it is not always necessary to trace the site of interest M. In this case, a button 15 for shifting to a site of interest tracking mode is provided, and whether the tracking of the site of interest M is performed or not is switched by the operator performing an ON/OFF operation of the button 15. The button 15 for switching whether or not the tracking of the site of interest M is performed may be a push button type operation button or an operation button displayed on the display unit on which a touch panel is mounted.

In the apparatus outline explained with reference to FIG. 1, although there is one display unit 8, for example, a plurality of display units may be provided according to the application so that an operation monitor with a touch panel is provided in addition to a confirmation monitor which only displays the fluoroscopic image. Additionally, when the site of interest tracking mode is turned off, the operator may perform the resetting of the region of interest R at an arbitrary timing during the X-ray fluoroscopy by touching the screen of the operation monitor while checking the site of interest M.

### [Reference Signs List]

1 X-ray tube
2 X-ray detector
3 Top plate
4 Subject
6 High voltage generation unit
7 Image generation unit
8 Display unit
9 Operation unit
10 Control unit
11 CPU
12 ROM
13 RAM
15 Button
21 Region of interest setting unit
22 Irradiation condition automatic adjustment unit
23 Site of interest tracking unit
24 Mode switching unit

## Claims

1. An X-ray fluoroscopic imaging apparatus, comprising:
an X-ray tube which generates X-rays to be radiated to a subject;
an X-ray detector which detects the X-rays radiated from the X-ray tube and passing through the subject;
an image generation unit which generates an X-ray image by processing a detection signal of the X-ray detector and displays the X-ray image on a display unit; and
a control unit having a processor, **characterized in that**,
the control unit comprises:
a region of interest setting unit which sets a region of interest on the X-ray image;
an irradiation condition automatic adjustment unit which calculates X-ray irradiation conditions on the basis of a set luminance of the region of interest; and
a site of interest tracking unit which tracks movement of a site of interest, wherein
the region of interest setting unit resets the region of interest according to the movement of the site of interest tracked by the site of interest tracking unit.

2. The X-ray fluoroscopic imaging apparatus according to claim 1, wherein:
the site of interest tracking unit extracts a feature amount of a region set as the region of interest, distinguishes the movement of the site of interest from a change in the feature amount and searches for a region having a feature amount closest to the feature amount in the X-ray image, and
the region of interest setting unit resets a region having a feature amount closest to the feature amount in the X-ray image identified by the site of interest tracking unit to the region of interest.

3. The X-ray fluoroscopic imaging apparatus according to claim 1, wherein:
the control unit comprises a storage unit which temporarily stores movement information when a display orientation of the X-ray image displayed on the display unit is changed,
the site of interest tracking unit calculates a movement position of the site of interest in the X-ray image on the basis of the movement information of the X-ray image acquired from the storage unit, and
the region of interest setting unit resets a region on the X-ray image including the site of interest, of which a position is calculated by the site of interest tracking unit, to the region of interest.

4. The X-ray fluoroscopic imaging apparatus according to claim 1, wherein:
the control unit comprises a storage unit which temporarily stores movement information when a position of the X-ray detector is changed,
the site of interest tracking unit calculates a movement position of the site of interest in the X-ray image on the basis of the movement information of the X-ray detector acquired from the storage unit, and
the region of interest setting unit resets a region on the X-ray image including the site of interest, of which a position is calculated by the site of interest tracking unit, to the region of interest.

5. The X-ray fluoroscopic imaging apparatus according to claim 1, wherein the control unit comprises a mode switching unit which receives an input from an operation button and switches between execution and stop of a tracking operation of the site of interest by the site of interest tracking unit.
